# DEMANDE DE BREVET EUROPEEN

(11) **EP 1 681 049 A1**
(43) Date de publication de la demande: **19.07.2006**
(21) Numéro de dépôt: 05290077.6
(22) Date de dépôt: 12.01.2005
(51) Int. Cl.: A61K 9/00, A61K 9/20

(54) **Composition pharmaceutique et forme galénique correspondante à délitement rapide en bouche, et procédé de fabrication de cette composition**

(71) Demandeur: Physica Pharma, 33600 Pessac (FR)
(72) Inventeur: Broussaud, Olivier, 33000 Bordeaux (FR); Pougnas, Jean-Luc, 33700 Merignac (FR); Calvet, Nicolas, 33000 Bordeaux (FR)
(74) Mandataire: Bolinches, Michel Jean-Marie

(57) **Abrégé**

La présente invention concerne une composition pharmaceutique utilisable pour constituer après compression une forme galénique à délitement rapide en bouche, une telle forme galénique et un procédé de fabrication de cette composition pharmaceutique.

Une composition selon l'invention, qui comprend des particules solides à base d'un mélange d'excipients comprenant au moins un diluant, est telle que ces particules sont revêtues d'un film à base d'une solution colloïdale aqueuse prévu pour pré-hydrater la composition, de sorte que les particules ainsi revêtues soient libres les unes par rapport aux autres dans la composition.

Le procédé de fabrication de cette composition comprend notamment la pulvérisation d'une solution colloïdale aqueuse comprenant une phase aqueuse et un système auto-émulsionnable sur ces particules solides, de telle manière que le rapport massique (solution colloïdale pulvérisable/particules destinées à recevoir la solution colloïdale) soit inférieur ou égal à 10 %.

## Description

La présente invention concerne une composition pharmaceutique pulvérulente utilisable pour constituer après compression une forme galénique à délitement rapide en bouche, une telle forme galénique et un procédé de fabrication de cette composition pharmaceutique.

Le développement de compositions pharmaceutiques destinées à constituer des formes galéniques solides qui, lorsqu'elles sont administrées dans la bouche de patients ayant des difficultés pour déglutir, tels que des enfants, des personnes âgées, des personnes mentalement déficientes ou non coopératives médicalement, sont aptes à se déliter (i.e. à se désagréger ou à se désintégrer) rapidement sans nécessiter un apport d'eau, suscite de nos jours un intérêt croissant.

Par « forme galénique solide à délitement rapide en bouche », on entend de manière connue, comme défini par la Pharmacopée Européenne, un comprimé non pelliculé (i.e. non enrobé) qui, lorsqu'il est introduit en bouche, se désintègre en moins de trois minutes avant d'être avalé. Le temps nécessaire à cette désintégration est communément appelé vitesse d'hydratation du comprimé.

Différentes formes galéniques solides à délitement rapide en bouche sont largement utilisées à ce jour. Il s'agit essentiellement de lyophilisats oraux, de comprimés effervescents oraux, ou encore de comprimés à forte teneur en agents de délitement pour l'obtention d'un délitement immédiat après introduction en bouche.

Les lyophilisats oraux ou « freeze drying tablets » représentent les compositions à délitement rapide les plus utilisées, notamment du fait qu'ils permettent d'assurer une désintégration éclair 10 secondes environ après leur administration en bouche, en raison de leur hygroscopicité et de leur porosité élevées. Néanmoins, ces lyophilisats présentent les inconvénients d'être très fragiles lors de leur manipulation en raison de leur friabilité élevée, d'être extrêmement sensibles à l'humidité atmosphérique et d'impliquer une technologie et un procédé de fabrication complexes et, par conséquent, des coûts de fabrication élevés. De ce fait, cette technologie est généralement retenue pour des principes actifs coûteux et faiblement dosés dans la composition pharmaceutique.

Les comprimés effervescents oraux sont constitués d'une composition pharmaceutique comprenant un couple effervescent, lequel entre en action dès son contact avec la salive, assurant ainsi le délitement complet du comprimé. Cependant, ces comprimés effervescents doivent nécessairement présenter une taille réduite afin de garantir un délitement en bouche inférieur à 3 minutes, de limiter la quantité de gaz émis et de limiter le mauvais goût en bouche qui les caractérise. Un autre inconvénient de ces comprimés effervescents est qu'ils sont très sensibles à l'humidité, ce qui implique l'utilisation d'un procédé de fabrication très particulier, typiquement en atmosphère contrôlée.

Les comprimés à forte teneur en agents de délitement sont par exemple décrits dans le document de Brevet US-A-5 464 632. Ce document divulgue une composition pharmaceutique qui comprend notamment, à titre d'agents de délitement associés à des agents de gonflement, une fraction massique élevée de carboxyméthylcellulose et d'une polyvinylpirrolidone réticulée et insoluble. Cette composition est obtenue par mélangeage d'un principe actif, utilisé sous forme de microcristaux ou de microgranules, avec un mélange d'excipients préalablement soumis à une granulation.

Cette fraction massique élevée d'agents de délitement dans les compositions selon ce document présente l'inconvénient de générer un goût de type crayeux et une sensation de sécheresse indésirable, dès l'introduction en bouche de ces comprimés les incorporant.

La plupart des excipients utilisés dans les compositions de comprimés à délitement rapide sont en général très solubles en milieu aqueux ou bien présentent un fort pouvoir de gonflement, typiquement dans le cas de l'utilisation d'agents de délitement.

Afin d'améliorer les sensations en bouche des comprimés, on a développé en particulier des compositions contenant majoritairement des excipients très solubles dans l'eau, tels que des polyols, du mannitol, du xylitol ou du sorbitol, la grande solubilité de ces excipients favorisant un délitement rapide des comprimés dès leur contact avec la salive. Néanmoins, l'utilisation de ces excipients, tels que des polyols, présente l'inconvénient d'augmenter les risques de collage lors de l'étape de compression et de ne pas réduire la sensibilité des comprimés à l'humidité ambiante.

Le document de Brevet WO-A-03/086361 préconise la mise en oeuvre d'une granulation par voie humide pour l'obtention d'une composition pharmaceutique destinée à former un comprimé à délitement rapide en bouche. Dans les exemples de ce document, cette granulation est réalisée via une pulvérisation d'une solution aqueuse de lauryl sulfate de sodium sur un mélange pulvérulent comprenant un principe actif dispersé dans des excipients.

Un inconvénient majeur de la granulation qui est mise en oeuvre dans ce document réside dans la densification des particules de poudre qu'elle implique, laquelle pénalise la pénétration de la salive à coeur du granulé obtenu même avec l'ajout de surfactant, ce qui va à l'encontre d'un délitement rapide des comprimés.

D'une manière connue, le développement de comprimés à délitement rapide en bouche doit prendre en compte un ensemble de paramètres physiques qui sont généralement contradictoires, incluant, pour les comprimés obtenus :
- une friabilité réduite et de préférence inférieure à 1 %, sinon la manipulation des comprimés peut devenir problématique tant lors de leur fabrication (risque d'endommagement des comprimés lors de l'étape de mise en emballage ou « blister », notamment) et lors de l'intervention de l'utilisateur final (risque d'écrasement des comprimés lors de l'extraction du « blister » et d'effritement des comprimés dans la main de l'utilisateur),
- une dureté ou résistance à la rupture qui est au contraire relativement élevée, étant typiquement d'au moins 15 Newtons, et
- une vitesse d'hydratation en bouche inférieure à trois minutes, cette vitesse étant généralement pénalisée en cas de dureté trop élevée des comprimés.

Un but de la présente invention est de remédier aux inconvénients précités, et ce but est atteint en ce que la Demanderesse vient de découvrir d'une manière surprenante que la pulvérisation d'une solution colloïdale aqueuse sur des particules solides à base d'un mélange d'excipients comprenant au moins un diluant, de telle manière que le rapport massique (solution colloïdale pulvérisable/ particules destinées à recevoir cette solution colloïdale) soit inférieur ou égal à 10 %, permet d'obtenir après séchage une composition pulvérulente qui est constituée desdites particules solides revêtues d'un film pré-hydratant à base de ladite solution colloïdale et qui est utilisable pour constituer après compression un comprimé présentant une vitesse d'hydratation (i.e. de délitement en bouche) nettement inférieure à 60 secondes et même inférieure à 30 secondes.

Selon l'invention, on notera que cette pulvérisation ne constitue en aucune manière une granulation (telle que les granulations par voie humide connues dans l'état de l'art), du fait que les particules revêtues du film pré-hydratant selon l'invention sont libres (i.e. n'adhèrent pas entre elles) et présentent une granulométrie et une densité apparente pratiquement inchangées, en raison du rapport massique précité relativement réduit qui se traduit par l'obtention en surface dudit film d'épaisseur réduite.

En effet, les granulés obtenus par des techniques de granulation sont caractérisés de manière connue par un agglomérat de particules collées les unes aux autres qui est recouvert d'une couche d'enrobage d'épaisseur relativement élevée, à la différence du film mince obtenu selon l'invention.

En d'autre termes, une composition pharmaceutique pulvérulente selon l'invention, qui est utilisable pour constituer après compression une forme galénique à délitement rapide en bouche et qui comprend lesdites particules solides, est telle que ces dernières sont revêtues dudit film pré-hydratant, de telle sorte que lesdites particules ainsi revêtues soient libres les unes par rapport aux autres dans ladite composition (i.e. ces particules ne sont pas collées les unes aux autres, du fait que la composition est dépourvue de tout liant).

Outre ce délitement accéléré en bouche, on notera que les comprimés selon l'invention présentent avantageusement :
- une dureté ou résistance à la rupture, mesurée selon la Pharmacopée Européenne, qui est supérieure à 15 Newtons et de préférence supérieure à 20 Newtons, et
- une friabilité, également mesurée selon la Pharmacopée Européenne, qui est inférieure à 1 %, ce qui permet de remédier à l'inconvénient précité relatif à la manipulation des comprimés à la fois lors de leur fabrication et lors de l'intervention de l'utilisateur final.

Cette composition pharmaceutique selon l'invention peut comprendre en outre au moins un principe actif à l'état dispersé dans ledit mélange d'excipients, cette dispersion du principe actif pouvant être réalisée après la pulvérisation (i.e. par mélangeage final dudit principe actif et desdites particules à base dudit mélange d'excipients ayant été revêtues dudit film) ou bien avant celle-ci (i.e. par mélangeage préalable dudit principe actif et desdites particules à base dudit mélange d'excipients non encore revêtues).

A titre de diluant(s) utilisable(s) dans ledit mélange d'excipients, on peut utiliser des diluants solubles dans un milieu aqueux de type eau ou salive ou aptes à gonfler dans ce milieu aqueux, le(s)dit(s) diluant(s) étant présent(s) dans ladite composition selon une fraction massique totale allant de 30 % à 90 % et de préférence allant de 50 à 85 %.

Ledit ou l'un au moins desdits diluants est avantageusement :
- un diluant soluble dans l'eau qui est choisi dans le groupe constitué par les dérivés de sucres, les dextrines et les maltodextrines et, encore plus avantageusement, il s'agit d'un dérivé de sucre choisi dans le groupe constitué par le lactose, le saccharose, le tréhalose, le mannitol, le sorbitol, l'érythritol, le maltitol, le lactitol et le fructose ; ou bien
- un diluant apte à gonfler en milieu aqueux qui est avantageusement choisi dans le groupe constitué par les dérivés d'amidons, les amidons prégélatinisés, les amidons modifiés (tels que les amidons de maïs et de pomme de terre ou les carboxyméthylamidons sodiques), les dérivés de gomme (tels que les gommes de guar, de xanthane ou de caroube), les dérivés de cellulose microcristalline (tels que les carboxyméthylcelluloses sodiques, les carboxyméthylcelluloses de calcium, les croscarmelloses), et les dérivés de la silice (tels que la silice colloïdale anhydre).

Selon une autre caractéristique de l'invention, la fraction massique de ladite solution colloïdale à l'état sec dans ladite composition appartient à un domaine allant de 0,01 % à 10 %, de préférence allant de 0,1 % à 5 % et, à titre encore plus préférentiel, allant de 0,5 % à 3 %.

A titre de solution colloïdale aqueuse utilisable pour constituer ledit film, on utilise une solution ou suspension de type huile dans eau ou bien eau dans huile (par exemple de type microémulsion) comprenant, d'une part, une phase aqueuse et, d'autre part, un système auto-émulsionnable, ce dernier comprenant en combinaison :
- au moins un composé filmogène hydrophile,
- au moins un composé amphiphile lubrifiant, et
- au moins un composé amphiphile humectant.

Par système auto-émulsionnable, on entend de manière connue un système permettant à l'émulsion de se former et de se reformer spontanément sans agitation mécanique ni apport d'énergie thermique. De cette manière, ladite solution colloïdale est apte à se reformer spontanément lors de la mise en contact avec la salive dudit film recouvrant lesdites particules solides.

A titre de composé(s) filmogène(s) hydrophile(s), on utilise un solide ou semi-solide pulvérulent à une température de 25° C qui est choisi dans le groupe constitué par les carbohydrates, les polyéthylène glycols, les polysaccharides, les dérivés de gommes (tels que les gommes de guar, de xanthane ou de caroube), les dérivés de polyglycérides et les mélanges de plusieurs de ces composés.

Avantageusement, ledit composé filmogène hydrophile selon l'invention est un carbohydrate choisi dans le groupe constitué par les maltodextrines, les dextrines, le sorbitol, le mannitol et le xylitol.

Selon une variante de l'invention, on utilise à titre de composé filmogène hydrophile un polyéthylène glycol de masse moléculaire moyenne en poids Mw allant de 1000 g/mol à 6000 g/mol.

Selon une autre variante de l'invention, on utilise à titre de composé filmogène hydrophile un dérivé de polyglycéride choisi dans le groupe constitué par les lauroyl macrogolglycérides ou les stéaroyl macrogolglycérides.

On notera que ledit composé filmogène hydrophile permet d'augmenter la vitesse d'hydratation de la composition pharmaceutique, tout en participant à la cohésion de celle-ci.

A titre de composé(s) amphiphile lubrifiant, on utilise avantageusement un composé choisi dans le groupe constitué par le docusate de sodium, le lauryl éther sulfate de sodium, le lauryl sulfate de sodium, les dérivés du glycérol (tels que le glycéryl béhénate et le glycéryl palmitostéarate), les polyéthylène glycols, la leucine, l'acide stéarique, le stéarate de magnésium, le sodium stéaryl fumarate et le benzoate de sodium.

A titre de composé(s) amphiphile humectant, on utilise un composé soluble ou dispersible en milieu aqueux qui est choisi dans le groupe constitué par les lipoaminoacides (tels que la capryloyl glycine et la lauroyl proline), les sorbitans et leur dérivés éthoxylés, les dérivés de lécithine, les dérivés de polyéthylène glycols, les dérivés de polyglycérides, les poloxamers (tels que les poloxamers « 188 » ou « 407 »), les sucres et les dérivés de sucres (tels que le palmitate de saccharose).

Avantageusement, ledit composé amphiphile humectant selon l'invention est un stéarate de sorbitan ou bien un polysorbate à titre de dérivé éthoxylé de sorbitan.

Selon une variante de l'invention, on utilise à titre de composé amphiphile humectant un dérivé de polyéthylène glycol choisi dans le groupe constitué par les cétomacrogol 1000 et les macrogol -25-cétostéaryl éther.

Selon une autre variante de l'invention, on utilise à titre de composé amphiphile humectant un dérivé de polyglycéride de type lauroyl macrogolglycéride ou stéaroyl macrogolglycéride, tel qu'un stéaroyl macrogol-32 glycéride ou un lauroyl macrogol-32 glycéride.

Ledit mélange d'excipients utilisé dans la composition selon l'invention peut également comprendre, en combinaison avec le(s)dit(s) principe(s) actif(s) et le(s)dit(s) diluant(s), un ou des agent(s) de cohésion (souvent appelés « glidant agents » par l'homme de l'art) tels que, à titre non limitatif, la cellulose microcristalline ou un de ses dérivés, une dextrine telle que la maltodextrine, un lactose, un dérivé de phosphate de calcium ou un dérivé d'amidon, le(s)dit(s) agent(s) de cohésion étant présent(s) dans ladite composition selon une fraction massique allant de 0,1 % à 40 %.

Ces agents de cohésion sont utilisés pour faciliter la fabrication des comprimés et maintenir la friabilité de ces derniers inférieure à 1 %. La fraction massique dans ladite composition selon l'invention du ou des agent(s) de cohésion peut être comprise entre 0,1 % et 40 % et, de préférence, cette fraction massique est inférieure à 20 %.

La composition pharmaceutique selon l'invention peut contenir des proportions variables de principe(s) actif(s), typiquement de 0,1 % à 50 %.

A titre exemplatif et nullement limitatif, en peut citer :
- des anti-ulcéreux, tels que Famotidine, Oméprazol, Lanzoprazol,
- des anti-émétiques, tels que Ondansetron, Granisetron, Dolasetron, Domperidone, Metoclopramide,
- des antihypertenseurs, tels que Enalapril, Losartan, Candesartan, Valsartan, Lisinopril, Ramipril, Doxazosin, Terazosin,
- des antihistaminiques, tels que Loratadine, Cetirizine,
- des anti-psychotiques, tels que Risperidone, Olanzapine, Quetiapine,
- des anti-dépresseurs, tels que Paroxetine, Fluoxetine, Mirtazapine,
- des analgésiques et anti-inflammatoires, tels que Piroxicam,
- des anti-hypercholestérolémiants, tels que Simvastatin, Lovastatin, Pravastatin,
- des anti-migraineux, tels que Zolmitriptan, Naratriptan, Rizatriptan, Sumatriptan,
- des anti-épileptiques, tels que Lamotrigine,
- des anti-Parkinsoniens, tels que Selegiline, Apomorphine,
- des anxiolytiques, tels que Diazepam, Lorazepam, Zolpidem,
- des anti-asthmatiques, tels que Zafirlukast, Montelukast,
- des agents du dysfonctionnement de l'érection, tels que Sildenafil,
- des anti-pyrétiques, tels que Paracétamol, l'acide acétylsalicylique, Ibuprofène, etc.

On notera que tous ces principes actifs peuvent être utilisés indifféremment sous leur forme base ou sous une forme de sels, d'hydrates, de solvates et d'isomères.

La composition pharmaceutique selon l'invention peut contenir en outre :
- des agents édulcorants, tels que du saccharinate de sodium ou de l'aspartame,
- des agents aromatisants, et/ou
- des agents lubrifiants, tels que le stéarate de magnésium, l'acide stéarique, le stéaryl fumarate de sodium, le talc, un PEG, ou des dérivés pulvérulents lipidiques comme par exemple le tribéhénate de glycérol.

Une forme galénique selon l'invention, qui présente un délitement rapide en bouche, comprend un comprimé non enrobé (i.e. non pelliculé) qui est constitué de ladite composition pharmaceutique telle que définie ci-dessus.

D'une manière générale, ces formes galéniques selon l'invention sont utilisables pour un traitement thérapeutique et/ou prophylactique d'un organisme humain ou animal.

Un procédé de fabrication selon l'invention de ladite composition pharmaceutique telle que définie ci-dessus comprend les étapes suivantes :
a) on prépare une solution colloïdale aqueuse huile dans eau ou eau dans huile comprenant, d'une part, une phase aqueuse et, d'autre part, un système auto-émulsionnable ;
b) on pulvérise la solution colloïdale obtenue en a) :
   (i) sur des particules solides constituées d'un principe actif dispersé dans un mélange d'excipients comprenant au moins un diluant, ou
   (ii) sur des particules solides seulement constituées dudit mélange d'excipients (i.e. constituées d'un pré-mélange non médicamenteux),
      de telle manière que le rapport massique (solution colloïdale pulvérisable/ particules destinées à recevoir ladite solution colloïdale) soit inférieur ou égal à 10 % ;
c) on sèche la solution colloïdale pulvérisée en b) pour l'obtention :
   - dans le cas (i), de ladite composition pharmaceutique constituée desdites particules solides comprenant ledit principe actif qui sont revêtues d'un film ; ou
   - dans le cas (ii), d'une composition intermédiaire constituée desdites particules solides d'excipients qui sont revêtues dudit film, puis
d) optionnellement, dans ce cas (ii), on disperse ledit principe actif dans ladite composition intermédiaire obtenue en c) pour l'obtention de ladite composition pharmaceutique.

De préférence, on choisit d'incorporer le(s)dit(s) principe(s) actif(s) à la composition pharmaceutique suite à la pulvérisation, comme indiqué à l'étape d), notamment dans le cas ou ledit ou l'un au moins desdits principes actifs est sensible à l'eau.

Selon une autre caractéristique de ce procédé selon l'invention, ledit système auto-émulsionnable comprend en combinaison, comme défini ci-dessus, le(s)dit(s) composé(s) filmogène(s) hydrophile(s), le(s)dit(s) composé(s) amphiphile(s) lubrifiant(s), et le(s)dit(s) composé(s) amphiphile(s) humectant(s).

Avantageusement, la solution colloïdale préparée à l'étape a) comprend une fraction massique dudit système auto-émulsionnable qui est inférieure ou égale à 10 %, et une fraction massique d'eau qui est égale ou supérieure à 90 %.

De préférence, la solution colloïdale obtenue à l'étape a) est chauffée à une température allant de 30° C à 70° C, pour la mise en oeuvre de l'étape b) à une telle température allant de 40° C à 70° C.

En effet, la Demanderesse a découvert que cette plage de températures permet avantageusement d'optimiser la pré-hydratation desdites particules solides par un mouillage et une tenue améliorés du film de solution colloïdale pulvérisé, en comparaison d'une pulvérisation réalisée à la température ambiante (par exemple 25°C).

Egalement à titre préférentiel, la viscosité de ladite solution colloïdale à pulvériser à l'étape b) appartient à un domaine allant de 2 mPa.s à 40 mPa.s à 50° C, et les gouttelettes pulvérisées présentent une taille moyenne allant sensiblement de 10 µm à 100 µm et, de préférence, allant de 50 µm à 70 µm. On notera que cette pulvérisation est en fait assimilable à une nébulisation dans le cas spécifique de gouttelettes dont la taille moyenne varie sensiblement de 10 à 50 µm.

On notera également que cette taille réduite des gouttelettes est particulièrement bien adaptée pour l'obtention dudit film mince pré-hydratant selon l'invention qui recouvre en surface lesdites particules solides sans les faire adhérer entre elles.

Selon une autre caractéristique de ce procédé selon l'invention, le rapport de la densité apparente de la composition obtenue à l'étape c) ou d) sur la densité apparente des particules solides avant pulvérisation est compris entre 1 et 1,2.

Selon une autre caractéristique de l'invention, la composition pharmaceutique ainsi obtenue présente, avant compression, une densité apparente qui, mesurée selon la Pharmacopée Européenne au moyen d'un voluménomètre de tassement (via 3 mesures de volumes apparents V₀, V₁₀ et V₅₀₀ - V₁₀ représentatives de l'aptitude au tassement), appartient à un domaine allant de 0,4 à 0,9 g /mL.

De plus, lesdites particules revêtues du film selon l'invention présentent, après pulvérisation et séchage, une augmentation de taille qui est inférieure ou égale à 20 %.

Les caractéristiques précitées de la présente invention, ainsi que d'autres, seront mieux comprises à la lecture de la description suivante de plusieurs exemples de réalisation de l'invention, donnés à titre illustratif et non limitatif.

### EXEMPLES :

### 1) Comprimé 1 « témoin » et comprimé 2 selon l'invention de type placebo :

### a) Préparation des comprimés :

On a préparé des comprimés « témoin » et selon l'invention à délitement rapide qui sont tous de type placebo, i.e. dépourvus de tout principe actif.

Ces comprimés placebo « témoin » et selon l'invention sont respectivement constitués de compositions 1 et 2 pulvérulentes présentant les formulations suivantes à l'état sec (en fractions massiques dans le tableau 1 a ci-après) :

**Tableau 1a :**

| COMPOSITIONS | 1 | 2 |
|---|---|---|
| Particules solides d'excipients : | | |
| PHARMABURST® (diluant à base de mannitol) | 76 % | 75.81 % |
| Arôme fraise | 1 % | 1 % |
| Aspartame | 1 % | 1 % |
| stéaryl fumarate de sodium | 2 % | 2 % |
| Cellulose microcristalline (agent de cohésion) | 20 % | 20 % |

| Film d'une solution colloïdale après séchage à base d'eau et du système auto-émulsionnable suivant : | | |
|---|---|---|
| Maltodextrine (composé hydrophile filmogène) | 0 % | 0,08 % |
| lauryl sulfate de sodium (composé amphiphile lubrifiant) | 0 % | 0,03 % |
| « GELUCIRE 44/14 » (composé amphiphile humectant) | 0 % | 0,08 % |

On notera que la composition 1 « témoin » n'a pas été pré-hydratée, contrairement à la composition 2 selon l'invention qui a été pré-hydratée par pulvérisation, sur les particules solides d'excipients, de la solution colloïdale aqueuse précitée, qui est de type huile dans eau et qui a été séchée en étuve après pulvérisation.

Le tableau 1b ci-après détaille la formulation utilisée pour l'obtention de cette solution colloïdale aqueuse (fractions massiques) :

**Tableau 1b :**

| | |
|---|---|
| Maltodextrine | 1,6 % |
| Lauryl sulfate de sodium | 0,6 % |
| « GELUCIRE 44/14 » | 1,6 % |
| Eau | 96,2 % |

Cette solution colloïdale filmogène a été chauffée à 50° C puis finement pulvérisée à cette même température selon un volume pulvérisé égal à 5 mL, étant précisé que sa viscosité à cette température était de 3 mPa.s.

De plus, les gouttelettes pulvérisées présentaient une taille moyenne allant sensiblement de 50 µm à 70 µm.

Cette composition 2 obtenue est telle que les particules d'excipients sont revêtues en surface d'un film d'épaisseur réduite, de telle sorte que les particules ainsi revêtues ne forment pas un granulé (i.e. ces particules n'adhèrent pas entre elles en dépit de la pulvérisation et présentent après cette dernière une taille sensiblement identique à leur taille initiale).

Le tableau 2 ci-après mentionne le volume apparent et la densité apparente des compositions 1 et 2 avant compression de celles-ci.

**Tableau 2 :**

| COMPOSITIONS | 1 | 2 |
|---|---|---|
| Volume apparent (cm³) | 190 | 186 |
| Densité apparente (g/cm³) | 0,526 | 0,537 |

On notera que l'absence de granulation, qui caractérise la pulvérisation mise en oeuvre pour l'obtention de la composition 2 selon l'invention, se traduit par une densité apparente de cette composition 2 avant compression qui est quasiment inchangée suite à la pulvérisation et au séchage, i.e. sensiblement égale à 0,526 g/cm³.

Chacun de ces comprimés « témoin » et selon l'invention, de type rond et plat, présente un diamètre de 12 mm, une épaisseur moyenne de 2,8 mm, une masse moyenne finale de 320 mg, une dureté moyenne de 21 Newtons et une friabilité de l'ordre de 0,45 %.

### b) Essais in vitro d'hydratation de ces comprimés :

On a procédé à des essais comparatifs de vitesse d'hydratation portant sur six comprimés 1 « témoin » et sur six comprimés 2 selon l'invention. Les conditions suivies pour ces essais étaient les suivantes :
- volume V de chaque comprimé : 317mm³, avec V= aire de la surface de la partie supérieure plane du comprimé x hauteur du comprimé), et
- volume d'eau déposé sur chaque comprimé : 320 mm³.

On a déposé 320 mm³ d'eau à la surface de chaque comprimé, puis on a mesuré le temps au bout duquel toute l'eau a disparu de la surface des comprimés. On a alors calculé la vitesse d'hydratation, cette vitesse correspondant en fait au temps nécessaire pour qu'un mm³ d'eau soit adsorbé par le comprimé correspondant.

Le tableau 3 ci-après présente les résultats moyens obtenus :

**Tableau 3 :**

| COMPOSITIONS | 1 | 2 |
|---|---|---|
| Temps d'absorption (en secondes) | 20 s | 33 s |
| Vitesse d'hydratation (pour 1 mm³) | 0,102 s/ mm³ | 0,0625 s/ mm³ |

Ces résultats montrent que les comprimés 2 selon l'invention, qui sont obtenus par pulvérisation de ladite solution colloïdale pré-hydratante sur lesdites particules d'excipients sans mettre en oeuvre une granulation, adsorbent l'eau environ 1,6 fois plus vite pour 1 mm³ que les comprimés 1 « témoin » qui sont dépourvus d'une telle solution colloïdale pré-hydratante, ce qui témoigne d'une vitesse d'hydratation nettement améliorée pour ces comprimés 2 selon l'invention.

### 2) Comprimés 3 selon l'invention :

### a) Préparation des comprimés 3 :

On a préparé des compositions pharmaceutiques 3 pulvérulentes selon l'invention d'une manière analogue à celle décrite pour les compositions 2 placebo selon l'invention du § 1) ci-dessus, excepté qu'après la pulvérisation de la solution colloïdale aqueuse sur un pré-mélange de particules solides d'excipients, on a procédé à une dispersion d'un principe actif dans les particules d'excipients revêtues du film de pulvérisation. Chaque composition 3 présente la formulation suivante à l'état sec (en fractions massiques dans le tableau 4a ci-après) :

**Tableau 4a :**

| COMPOSITION | 3 |
|---|---|
| Pré-mélange de particules d'excipients revêtu par pulvérisation : | |
| PHARMABURST® (diluant à base de mannitol) | 78 % |
| Arôme menthe | 1 % |
| Aspartame | 1 % |
| « PRUV » (lubrifiant anti-collant pour la mise en oeuvre) | 4 % |
| cellulose microcristalline « AVICEL Ph 200 » (agent de cohésion) | 10 % |

| Film d'une solution colloïdale selon l'invention après séchage à base d'eau et du système auto-émulsionnable suivant : | |
|---|---|
| « PEG 4000 » (composé hydrophile filmogène) | 2 % |
| lauryl éther sulfate de sodium (composé amphiphile lubrifiant) | 0,5 % |
| « GELUCIRE 44/14 » (composé amphiphile humectant) | 0,5 % |
| Glycérine | 2 % |
| Rispéridone (principe actif) | 1 % |

Le tableau 4b ci-après détaille la formulation utilisée pour l'obtention de cette solution colloïdale aqueuse (fractions massiques) :

**Tableau 4b :**

| | |
|---|---|
| « PEG 4000 » | 20 % |
| Lauryl éther sulfate de sodium | 5 % |
| « GELUCIRE 44/14 » | 5 % |
| Glycérine | 20 % |
| Eau | 50 % |

Cette solution colloïdale filmogène a été chauffée à 50° C puis pulvérisée en fines gouttelettes sur les particules d'excipients en mouvement à cette même température, selon un volume pulvérisé égal à 10 mL.

On a produit ces fines gouttelettes via une buse de pulvérisation classique de type à cône creux. Des essais ont montré que la durée nécessaire et suffisante pour pulvériser ces 10 mL de solution colloïdale sur les particules d'excipients était de 3 minutes.

Le protocole opératoire suivi était le suivant :
- on a agité le pré-mélange de particules d'excipients à revêtir (ce pré-mélange étant dépourvu de principe actif), via un mélangeur planétaire avec ou sans double enveloppe ; puis
- on a pulvérisé la solution colloïdale filmogène pendant 3 minutes, sur ce pré-mélange d'excipients en mouvement ; puis
- on a mesuré l'humidité résiduelle des particules ainsi revêtues, qui était de l'ordre de 7 % ; puis
- on a séché pendant 5 heures en étuve, à une température de 50° C, les particules du pré-mélange revêtues de cette solution colloïdale, suite à la pulvérisation ; puis
- on a mesuré l'humidité résiduelle des particules revêtues et séchées, qui était de l'ordre de 2,6 % ; puis
- on a procédé à la dispersion par mélangeage du principe actif, en lubrifiant l'ensemble.

On notera que l'absence de granulation, qui caractérise la pulvérisation mise en oeuvre pour l'obtention de la composition 3, se traduit par une densité apparente de cette composition 3 avant compression qui est inchangée suite à la pulvérisation et au séchage.

On notera également que l'on pourrait utiliser pratiquement le même protocole, pour l'obtention d'une composition dans laquelle la pulvérisation de la solution colloïdale serait directement effectuée sur les particules solides incorporant le principe actif en plus des excipients.

Après compression, on a obtenu des comprimés 3 selon l'invention à délitement rapide en bouche, qui contiennent chacun 1 mg de Rispéridone à titre de principe actif.

Chaque comprimé 3 présente une masse finale de 100 mg, une dureté moyenne de 21 Newtons, une épaisseur de 2,2 mm et une friabilité de l'ordre de 0,45 %.

### b) Essais in vivo d'hydratation de ces comprimés 3 :

On a procédé à des essais d'hydratation sur six individus humains. On a déterminé la vitesse d'hydratation de ces comprimés 3 selon l'invention, définie ici comme étant la durée nécessaire pour obtenir la désintégration ou délitement en bouche de ces comprimés par l'action de la salive, en réalisant une moyenne sur les six individus. On a ainsi obtenu pour ces comprimés 3 une vitesse d'hydratation de 25 secondes.

### 3) Comprimés 4 selon l'invention :

### a) Préparation des comprimés 4 :

On a préparé des compositions pharmaceutiques 4 pulvérulentes selon l'invention d'une manière analogue à celle décrite au § 2) ci-dessus (i.e. après la pulvérisation de la solution colloïdale aqueuse sur les particules solides d'excipients, on a procédé à une dispersion d'un principe actif dans les particules d'excipients revêtues du film de pulvérisation). Chaque composition 4 présente la formulation suivante à l'état sec (en fractions massiques dans le tableau 5 ci-après) :

**Tableau 5 :**

| COMPOSITION | 4 |
|---|---|
| Pré-mélange de particules d'excipients revêtu par pulvérisation : | |
| Mannitol (diluant) | 81,4 % |
| Arôme menthe | 1 % |
| Aspartame | 1 % |
| « PRUV » (lubrifiant anti-collant pour la mise en oeuvre) | 4 % |
| cellulose microcristalline « AVICEL Ph 200 » (agent de cohésion) | 10 % |

| Film d'une solution colloïdale selon l'invention après séchage à base d'eau et du système auto-émulsionnable suivant : | |
|---|---|
| « PEG 4000 » (composé hydrophile filmogène) | 0,1 % |
| « PEG 400 » (composé hydrophile filmogène) | 0,4 % |
| Lécithine alimentaire (composé amphiphile lubrifiant) | 1 % |
| « GELUCIRE 44/14 » (composé amphiphile humectant) | 0,5 % |
| Rispéridone (principe actif) | 0,6 % |

On notera que l'absence de granulation, qui caractérise la pulvérisation mise en oeuvre pour l'obtention de la composition 4, se traduit par une densité apparente de cette composition 4 avant compression qui est inchangée suite à la pulvérisation et au séchage.

Après compression, on a obtenu des comprimés 4 selon l'invention à délitement rapide en bouche, qui contiennent chacun 1 mg de Rispéridone à titre de principe actif.

Chaque comprimé 4 présente une masse finale de 166 mg, une dureté moyenne de 22 Newtons, une épaisseur de 2,08 mm et une friabilité de l'ordre de 0,75 %.

### b) Essais in vivo d'hydratation de ces comprimés 4 :

On a procédé à des essais d'hydratation sur six individus humains, en procédant comme décrit au § 2) ci-dessus. On a ainsi obtenu pour ces comprimés 4 une vitesse d'hydratation de 18 secondes.

### 4) Comprimés 5 selon l'invention :

### a) Préparation des comprimés 5 :

On a préparé des compositions pharmaceutiques 5 pulvérulentes selon l'invention d'une manière analogue à celle décrite au § 2) ci-dessus. Chaque composition 5 présente la formulation suivante à l'état sec (en fractions massiques dans le tableau 6 ci-après) :

**Tableau 6 :**

| COMPOSITION | 5 |
|---|---|
| Pré-mélange de particules d'excipients revêtu par pulvérisation : | |
| PHARMABURST® (diluant à base de mannitol) | 67,87 % |
| Arôme menthe | 1 % |
| Aspartame | 1 % |
| « PRUV » (lubrifiant anti-collant pour la mise en oeuvre) | 2 % |
| cellulose microcristalline « AVICEL Ph 200 » (agent de cohésion) | 10 % |

| Film d'une microémulsion aqueuse selon l'invention après séchage à base d'eau et du système auto-émulsionnable suivant : | |
|---|---|
| « GELUCIRE 44/14 » (composé hydrophile filmogène) | 8 % |
| Stéarate de magnésium (composé amphiphile lubrifiant) | 2 % |
| Lauryl sulfate de sodium (composé amphiphile humectant) | 2 % |
| Ordansetron (principe actif) | 6,13 % |

On notera que l'absence de granulation, qui caractérise la pulvérisation mise en oeuvre pour l'obtention de la composition 5, se traduit par une densité apparente de cette composition 5 avant compression qui est inchangée suite à la pulvérisation et au séchage.

Après compression, on a obtenu des comprimés 5 selon l'invention à délitement rapide en bouche, qui contiennent chacun 4 mg d'Ordansetron à titre de principe actif.

Chaque comprimé 5 présente une masse finale de 65 mg, une dureté moyenne de 19 Newtons, une épaisseur de 2,08 mm et une friabilité de l'ordre de 0,58 %.

### b) Essais in vivo d'hydratation de ces comprimés 5 :

On a procédé à des essais d'hydratation sur six individus humains, en procédant comme décrit au § 2) ci-dessus. On a ainsi obtenu pour ces comprimés 5 une vitesse d'hydratation de 22 secondes.

### 5) Comprimés 6 selon l'invention :

### a) Préparation des comprimés 6 :

On a préparé des compositions pharmaceutiques 6 pulvérulentes selon l'invention d'une manière analogue à celle décrite au § 2) ci-dessus. Chaque composition 6 présente la formulation suivante à l'état sec (en fractions massiques dans le tableau 7 ci-après) :

**Tableau 7 :**

| COMPOSITION | 6 |
|---|---|
| Pré-mélange de particules d'excipients revêtu par pulvérisation : | |
| Erythritol (diluant) | 59,87 % |
| Arôme fraise | 1,5 % |
| Aspartame | 1 % |
| « PRUV » (lubrifiant anti-collant pour la mise en oeuvre) | 2 % |
| cellulose microcristalline « AVICEL Ph 200 » (agent de cohésion) | 20 % |

| Film d'une microémulsion aqueuse selon l'invention après séchage à base d'eau et du système auto-émulsionnable suivant : | |
|---|---|
| « PEG 1500 » (composé hydrophile filmogène) | 1 % |
| « GELUCIRE 50/13 » (composé amphiphile humectant) | 5,5 % |
| Stéarate de magnésium (composé amphiphile lubrifiant) | 2 % |
| Lauryl sulfate de sodium (composé amphiphile lubrifiant) | 1 % |
| Lopéramide (principe actif) | 6,13 % |

On notera que l'absence de granulation, qui caractérise la pulvérisation mise en oeuvre pour l'obtention de la composition 6, se traduit par une densité apparente de cette composition 6 avant compression qui est inchangée suite à la pulvérisation et au séchage.

Après compression, on a obtenu des comprimés 6 selon l'invention à délitement rapide en bouche, qui contiennent chacun 2 mg de Lopéramide à titre de principe actif.

Chaque comprimé 6 présente une masse finale de 166 mg, une dureté moyenne de 22 Newtons, une épaisseur de 2,08 mm et une friabilité de l'ordre de 0,75 %.

### b) Essais in vivo d'hydratation de ces comprimés 6 :

On a procédé à des essais d'hydratation sur six individus humains, en procédant comme décrit au § 2) ci-dessus. On a ainsi obtenu pour ces comprimés 6 une vitesse d'hydratation de 19 secondes.

### 6) Comprimés 7 selon l'invention :

### a) Préparation des comprimés 7 :

On a préparé des compositions pharmaceutiques 7 pulvérulentes selon l'invention d'une manière analogue à celle décrite au § 2) ci-dessus. Chaque composition 7 présente la formulation suivante à l'état sec (en fractions massiques dans le tableau 8 ci-après) :

**Tableau 8 :**

| COMPOSITION | 7 |
|---|---|
| Pré-mélange de particules d'excipients revêtu par pulvérisation : | |
| PHARMABURST® (diluant à base de mannitol) | 50,17 % |
| Arôme menthe | 1 % |
| Aspartame | 2 % |
| « PRUV » (lubrifiant anti-collant pour la mise en oeuvre) | 2 % |
| cellulose microcristalline « AVICEL Ph 200 » (agent de cohésion) | 10 % |

| Film d'une microémulsion aqueuse selon l'invention après séchage à base d'eau et du système auto-émulsionnable suivant : | |
|---|---|
| « PEG 1000 » (composé hydrophile filmogène) | 0,8 % |
| Lauryl sulfate de sodium (composé amphiphile humectant) | 0,5 % |
| Tribéhénate de glycérol (composé amphiphile lubrifiant) | 0,2 % |
| Paracétamol (principe actif) | 33,33 % |

On notera que l'absence de granulation, qui caractérise la pulvérisation mise en oeuvre pour l'obtention de la composition 7, se traduit par une densité apparente de cette composition 7 avant compression qui est inchangée suite à la pulvérisation et au séchage.

Après compression, on a obtenu des comprimés 7 selon l'invention à délitement rapide en bouche, qui contiennent chacun 500 mg de Paracétamol à titre de principe actif.

Chaque comprimé 7 présente une masse finale de 1500 mg, une dureté moyenne de 25 Newtons, une épaisseur de 3,5 mm et une friabilité de l'ordre de 0,75 %.

### b) Essais in vivo d'hydratation de ces comprimés 7 :

On a procédé à des essais d'hydratation sur six individus humains, en procédant comme décrit au § 2) ci-dessus. On a ainsi obtenu pour ces comprimés 7 une vitesse d'hydratation de 30 secondes.

### 7) Comprimés 8 selon l'invention :

### a) Préparation des comprimés 8 :

On a préparé des compositions pharmaceutiques 8 pulvérulentes selon l'invention d'une manière analogue à celle décrite au § 2) ci-dessus. Chaque composition 8 présente la formulation suivante à l'état sec (en fractions massiques dans le tableau 9 ci-après) :

**Tableau 9 :**

| COMPOSITION | 8 |
|---|---|
| Pré-mélange de particules d'excipients revêtu par pulvérisation : | |
| Mannitol (diluant) | 61,65 % |
| Arôme menthe | 1 % |
| Aspartame | 2 % |
| « PRUV » (lubrifiant anti-collant pour la mise en oeuvre) | 2 % |
| cellulose microcristalline « AVICEL Ph 200 » (agent de cohésion) | 10 % |
| « PEG 1000 » | 0,3 % |
| Stéarate de magnésium | 2 % |

| Film d'une solution colloïdale selon l'invention après séchage à base d'eau et du système auto-émulsionnable suivant : | |
|---|---|
| « Poloxamer 188 » (composé hydrophile filmogène) | 0,4 % |
| Lauryl sulfate de sodium (composé amphiphile lubrifiant) | 0,15 % |
| « GELUCIRE 44/14 » (composé amphiphile humectant) | 0,5 % |
| Paracétamol (principe actif) | 20 % |

On notera que l'absence de granulation, qui caractérise la pulvérisation mise en oeuvre pour l'obtention de la composition 8, se traduit par une densité apparente de cette composition 8 avant compression qui est inchangée suite à la pulvérisation et au séchage.

Après compression, on a obtenu des comprimés 8 selon l'invention à délitement rapide en bouche, qui contiennent chacun 500 mg de Paracétamol à titre de principe actif.

Chaque comprimé 8 présente une masse finale de 2500 mg, une dureté moyenne de 29 Newtons, une épaisseur de 3 mm et une friabilité de l'ordre de 0,75 %.

### b) Essais in vivo d'hydratation de ces comprimés 8 :

On a procédé à des essais d'hydratation sur six individus humains, en procédant comme décrit au § 2) ci-dessus. On a ainsi obtenu pour ces comprimés 8 une vitesse d'hydratation de 28 secondes.

### 8) Comprimés 9 selon l'invention :

### a) Préparation des comprimés 9 :

On a préparé des compositions pharmaceutiques 9 pulvérulentes selon l'invention d'une manière analogue à celle décrite au § 2) ci-dessus. Chaque composition 9 présente la formulation suivante à l'état sec (en fractions massiques dans le tableau 10 ci-après) :

**Tableau 10 :**

| COMPOSITION | 9 |
|---|---|
| Pré-mélange de particules d'excipients revêtu par pulvérisation : | |
| PHARMABURST® (diluant à base de mannitol) | 79,25 % |
| Arôme menthe | 1 % |
| Aspartame | 2 % |
| « PRUV » (lubrifiant anti-collant pour la mise en oeuvre) | 4 % |
| cellulose microcristalline « AVICEL Ph 200 » (agent de cohésion) | 10 % |

| Film d'une microémulsion aqueuse selon l'invention après séchage à base d'eau et du système auto-émulsionnable suivant : | |
|---|---|
| « PEG 1500 » (composé hydrophile filmogène) | 0,5 % |
| « Poloxamer 188 » (composé amphiphile humectant) | 0,5 % |
| « PRECIROL ATO 5 » (composé amphiphile lubrifiant) | 0,25 % |
| Zolmitriptan (principe actif) | 2,5 % |

On notera que l'absence de granulation, qui caractérise cette pulvérisation, se traduit par une densité apparente de cette composition 9 avant compression qui est inchangée suite à la pulvérisation et au séchage.

Après compression, on a obtenu des comprimés 9 selon l'invention à délitement rapide en bouche, qui contiennent chacun 2,5 mg de Zolmitriptan à titre de principe actif. Chaque comprimé 9 présente une masse finale de 100 mg, une dureté moyenne de 21 Newtons, une épaisseur de 2,07 mm et une friabilité de l'ordre de 0,7 %.

### b) Essais in vivo d'hydratation de ces comprimés 9 :

On a procédé à des essais d'hydratation sur six individus humains, en procédant comme décrit au § 2) ci-dessus. On a ainsi obtenu pour ces comprimés 9 une vitesse d'hydratation de 28 secondes.

### 9) Comprimés 10 selon l'invention :

### a) Préparation des comprimés 10 :

On a préparé des compositions pharmaceutiques 10 pulvérulentes selon l'invention d'une manière analogue à celle décrite au § 2) ci-dessus. Chaque composition 10 présente la formulation suivante à l'état sec (en fractions massiques dans le tableau 11 ci-après) :

**Tableau 11 :**

| COMPOSITION | 10 |
|---|---|
| Pré-mélange de particules d'excipients revêtu par pulvérisation : | |
| Mannitol (diluant) | 32,77 % |
| Sorbitol (diluant) | 15 % |
| Arôme menthe | 1 % |
| Aspartame | 2 % |
| cellulose microcristalline « AVICEL Ph 200 » (agent de cohésion) | 10 % |

| Film d'une microémulsion aqueuse selon l'invention après séchage à base d'eau et du système auto-émulsionnable suivant : | |
|---|---|
| « PEG 1500 » (composé hydrophile filmogène) | 3 % |
| « Poloxamer 188 » (composé amphiphile humectant) | 0,6 % |
| « Stéarate de magnésium » (composé amphiphile lubrifiant) | 2 % |
| « GELUCIRE 44/14 » (composé amphiphile humectant) | 0,3 % |
| Ibuprofène (principe actif) | 33,33 % |

On notera que l'absence de granulation, qui caractérise cette pulvérisation, se traduit par une densité apparente de cette composition 10 avant compression qui est inchangée suite à la pulvérisation et au séchage.

Après compression, on a obtenu des comprimés 10 selon l'invention à délitement rapide en bouche, qui contiennent chacun 200 mg d'Ibuprofène à titre de principe actif. Chaque comprimé 10 présente une masse finale de 600 mg, une dureté moyenne de 21 Newtons, une épaisseur de 2,07 mm et une friabilité de l'ordre de 0,7 %.

### b) Essais in vivo d'hydratation de ces comprimés 10 :

On a procédé à des essais d'hydratation sur six individus humains, en procédant comme décrit au § 2) ci-dessus. On a ainsi obtenu pour ces comprimés 10 une vitesse d'hydratation de 28 secondes.

## Revendications

1. Composition pharmaceutique pulvérulente utilisable pour constituer après compression une forme galénique à délitement rapide en bouche, ladite composition comprenant des particules solides à base d'un mélange d'excipients qui comprend au moins un diluant, **caractérisée en ce que** lesdites particules sont revêtues d'un film à base d'une solution colloïdale aqueuse qui est prévu pour pré-hydrater ladite composition, de telle sorte que lesdites particules ainsi revêtues soient libres les unes par rapport aux autres dans ladite composition.

2. Composition pharmaceutique selon la revendication 1, **caractérisée en ce que** la fraction massique de ladite solution colloïdale à l'état sec dans ladite composition appartient à un domaine allant de 0,01 % à 10 %.

3. Composition pharmaceutique selon la revendication 2, **caractérisée en ce que** la fraction massique de ladite solution colloïdale à l'état sec dans ladite composition appartient à un domaine allant de 0,1 % à 5 %.

4. Composition pharmaceutique selon la revendication 3, **caractérisée en ce que** la fraction massique de ladite solution colloïdale à l'état sec dans ladite composition appartient à un domaine allant de 0,5 % à 3%.

5. Composition pharmaceutique selon une des revendications précédentes, **caractérisée en ce que** ladite solution colloïdale comprend un système auto-émulsionnable comprenant en combinaison :
- au moins un composé filmogène hydrophile,
- au moins un composé amphiphile lubrifiant, et
- au moins un composé amphiphile humectant.

6. Composition pharmaceutique selon la revendication 5, **caractérisée en ce que** ledit composé filmogène hydrophile est un solide pulvérulent à une température de 25° C qui est choisi dans le groupe constitué par les carbohydrates, les polyéthylène glycols, les polysaccharides, les dérivés de gommes, les dérivés de polyglycérides et les mélanges de plusieurs de ces composés.

7. Composition pharmaceutique selon la revendication 6, **caractérisée en ce que** ledit composé filmogène hydrophile est un carbohydrate choisi dans le groupe constitué par les maltodextrines, les dextrines, le sorbitol, le mannitol et le xylitol.

8. Composition pharmaceutique selon la revendication 6, **caractérisée en ce que** ledit composé filmogène hydrophile est un polyéthylène glycol de masse moléculaire moyenne en poids Mw allant de 1000 g/mol à 6000 g/mol.

9. Composition pharmaceutique selon la revendication 6, **caractérisée en ce que** ledit composé filmogène hydrophile est un dérivé de polyglycéride choisi dans le groupe constitué par les lauroyl macrogolglycérides ou les stéaroyl macrogolglycérides.

10. Composition pharmaceutique selon une des revendications 5 à 9, **caractérisée en ce que** ledit composé amphiphile lubrifiant est choisi dans le groupe constitué par le docusate de sodium, le lauryl éther sulfate de sodium, le lauryl sulfate de sodium, les dérivés du glycérol, les polyéthylène glycols, la leucine, l'acide stéarique, le stéarate de magnésium, le sodium stéaryl fumarate et le benzoate de sodium.

11. Composition pharmaceutique selon une des revendications 5 à 10, **caractérisée en ce que** ledit composé amphiphile humectant est choisi dans le groupe constitué par les lipoaminoacides, les sorbitans et leur dérivés éthoxylés, les dérivés de lécithine, les dérivés de polyéthylène glycols, les dérivés de polyglycérides, les poloxamers, les sucres et les dérivés de sucres.

12. Composition pharmaceutique selon la revendication 11, **caractérisée en ce que** ledit composé amphiphile humectant est un poloxamer, tel qu'un poloxamer « 188 ».

13. Composition pharmaceutique selon la revendication 11, **caractérisée en ce que** ledit composé amphiphile humectant est un dérivé de polyéthylène glycol choisi dans le groupe constitué par les cétomacrogol 1000 et les macrogol -25-cétostéaryl éther.

14. Composition pharmaceutique selon la revendication 11, **caractérisée en ce que** ledit composé amphiphile humectant est un dérivé de polyglycéride de type lauroyl macrogolglycéride ou stéaroyl macrogolglycéride.

15. Composition pharmaceutique selon une des revendications précédentes, **caractérisée en ce que** ledit ou chaque diluant est soluble dans un milieu aqueux de type eau ou salive ou est apte à gonfler dans ledit milieu aqueux, le(s)dit(s) diluant(s) étant présent(s) dans ladite composition selon une fraction massique totale allant de 30 % à 90 %.

16. Composition pharmaceutique selon la revendication 15, **caractérisée en ce que** ledit ou l'un au moins desdits diluants est un diluant soluble dans l'eau qui est choisi dans le groupe constitué par les dérivés de sucres, les dextrines et les maltodextrines.

17. Composition pharmaceutique selon la revendication 16, **caractérisée en ce que** ledit ou l'un au moins desdits diluants comprend un dérivé de sucres choisi dans le groupe constitué par le lactose, le saccharose, le tréhalose, le mannitol, le sorbitol, l'érythritol, le maltitol, le lactitol et le fructose.

18. Composition pharmaceutique selon la revendication 15, **caractérisée en ce que** ledit ou l'un au moins desdits diluants est un diluant apte à gonfler en milieu aqueux qui est choisi dans le groupe constitué par les dérivés d'amidons, les amidons prégélatinisés, les amidons modifiés, les dérivés de gomme, les dérivés de cellulose microcristalline, et les dérivés de la silice.

19. Composition pharmaceutique selon une des revendications précédentes, **caractérisée en ce que** ledit mélange d'excipients comprend en outre au moins un agent de cohésion, tel que la cellulose microcristalline ou un de ses dérivés, une dextrine, un lactose, un dérivé de phosphate de calcium ou un dérivé d'amidon, le(s)dit(s) agent(s) de cohésion étant présent(s) dans ladite composition selon une fraction massique allant de 0,1 % à 40 %.

20. Composition pharmaceutique selon une des revendications précédentes, **caractérisée en ce qu'**elle présente avant compression une densité apparente, mesurée selon la Pharmacopée Européenne, qui appartient à un domaine allant de 0,4 à 0,9 g /mL.

21. Composition pharmaceutique selon une des revendications précédentes, **caractérisée en ce qu'**elle comprend au moins un principe actif à l'état dispersé dans ledit mélange d'excipients.

22. Forme galénique à délitement rapide en bouche et comprenant un comprimé non enrobé, **caractérisée en ce que** ledit comprimé est constitué d'une composition pharmaceutique selon une des revendications 1 à 21.

23. Forme galénique selon la revendication 22, **caractérisé en ce que** ledit comprimé présente une friabilité, mesurée conformément à la Pharmacopée Européenne, qui est inférieure à 1 %.

24. Forme galénique selon la revendication 22 ou 23, **caractérisé en ce que** ledit comprimé présente une résistance à la rupture, mesurée conformément à la Pharmacopée Européenne, qui est supérieure à 15 Newtons.

25. Procédé de fabrication d'une composition pharmaceutique selon une des revendications 1 à 21, **caractérisé en ce qu'**il comprend les étapes suivantes :
a) on prépare une solution colloïdale aqueuse de type huile dans eau ou eau dans huile comprenant, d'une part, une phase aqueuse et, d'autre part, un système auto-émulsionnable ;
b) on pulvérise la solution colloïdale obtenue en a) :
(i) sur des particules solides constituées d'un principe actif dispersé dans un mélange d'excipients comprenant au moins un diluant, ou
(ii) sur des particules solides seulement constituées dudit mélange d'excipients,
de telle manière que le rapport massique (solution colloïdale pulvérisable/ particules destinées à recevoir ladite solution colloïdale) soit inférieur ou égal à 10 % ;
c) on sèche la solution colloïdale pulvérisée en b) pour l'obtention :
- dans le cas (i), de ladite composition pharmaceutique constituée desdites particules solides comprenant ledit principe actif qui sont revêtues d'un film ; ou
- dans le cas (ii), d'une composition intermédiaire constituée desdites particules solides d'excipients qui sont revêtues dudit film, puis
d) optionnellement, dans ce cas (ii), on disperse ledit principe actif dans ladite composition intermédiaire obtenue en c) pour l'obtention de ladite composition pharmaceutique.

26. Procédé selon la revendication 25, **caractérisé en ce que** ledit système auto-émulsionnable comprend en combinaison :
- au moins un composé filmogène hydrophile, tel qu'un solide pulvérulent à une température de 25° C qui est choisi dans le groupe constitué par les carbohydrates, les polyéthylène glycols, les dérivés de polyglycérides et les mélanges de plusieurs de ces composés,
- au moins un composé amphiphile lubrifiant, tel qu'un composé choisi dans le groupe constitué par le docusate de sodium, le lauryl éther sulfate de sodium, le lauryl sulfate de sodium, les dérivés du glycérol, les polyéthylène glycols, la leucine, l'acide stéarique, le stéarate de magnésium, le sodium stéaryl fumarate et le benzoate de sodium, et
- au moins un composé amphiphile humectant, tel qu'un composé choisi dans le groupe constitué par les lipoaminoacides, les sorbitans et leur dérivés éthoxylés, les dérivés de lécithine, les dérivés de polyéthylène glycols, les dérivés de polyglycérides, les poloxamers, les sucres et les dérivés de sucres.

27. Procédé selon la revendication 25 ou 26, **caractérisé en ce que** ladite solution colloïdale préparée à l'étape a) comprend :
- une fraction massique dudit système auto-émulsionnable inférieure ou égale à 10 %, et
- une fraction massique d'eau égale ou supérieure à 90 %.

28. Procédé selon une des revendications 25 à 27, **caractérisé en ce que** l'étape b) est mise en oeuvre à une température allant de 40° C à 70° C.

29. Procédé selon une des revendications 25 à 28, **caractérisé en ce que** la viscosité à 50° C de ladite solution colloïdale à pulvériser à l'étape b) appartient à un domaine allant de 2 mPa.s à 40 mPa.s.

30. Procédé selon une des revendications 25 à 29, **caractérisé en ce que** le rapport de la densité apparente de la composition obtenue à l'étape c) ou d) sur la densité apparente des particules solides avant pulvérisation est compris entre 1 et 1,2.
